# EUROPEAN PATENT SPECIFICATION

(11) **EP 3 863 595 B1**
(45) Date of publication and mention of the grant of the patent: **01.05.2024**
(21) Application number: 19802304.6
(22) Date of filing: 10.10.2019
(51) Int. Cl.: A61K 8/34, A61K 8/37, A61K 8/891, A61K 8/92, A61Q 1/04

(54) **LIP COSMETIC COMPOSITION**
LIPPENSTIFT
COMPOSITION DE ROUGE A LEVRES

(30) Priority: 10.10.2018 JP 2018191852
(43) Date of publication of application: 18.08.2021
(73) Proprietor: Chanel Parfums Beauté, 92200 Neuilly-sur-Seine (FR)
(72) Inventor: ICHIKAWA, Chikako, Funabashi-shi, Chiba 273-0045 (JP); YAMAKI, Hideyuki, Funabashi-shi, Chiba 273-0045 (JP); KUROMIYA, Tomomi, Funabashi-shi, Chiba 273-0045 (JP); ITO, Hisao, Funabashi-shi, Chiba 273-0045 (JP)
(74) Representative: Plasseraud IP
(86) International application number: PCT/JP2019/041233
(87) International publication number: WO 2020/075875

(56) References cited:
- DATABASE GNPD [Online] MINTEL; 7 September 2018 (2018-09-07), anonymous: "So Kissable Lipstick", XP055657714, retrieved from www.gnpd.com Database accession no. 5932067
- DATABASE GNPD [Online] MINTEL; 28 July 2017 (2017-07-28), anonymous: "Plush Up Lip Gelato", XP055657716, retrieved from www.gnpd.com Database accession no. 4957291
- DATABASE GNPD [Online] MINTEL; 18 July 2015 (2015-07-18), anonymous: "Long Keep Rouge Lipstick", XP055657717, retrieved from www.gnpd.com Database accession no. 3343631
- DATABASE GNPD [Online] MINTEL; 21 September 2010 (2010-09-21), anonymous: "Lipclick Full Color Lipstick", XP055657719, retrieved from www.gnpd.com Database accession no. 1390892
- DATABASE GNPD [Online] MINTEL; 13 January 2020 (2020-01-13), anonymous: "Allure Shine Lustrous Lip Plumper", XP055657713, Database accession no. 7129299

## Description

### TECHNICAL FIELD

The present invention relates to a lip cosmetic composition and use thereof.

### BACKGROUND ART

A lip cosmetic such as a lipstick worn on lips sometimes transfers and leaves color on a place such as a cup where the lips make contact. Color transfer not only stains the place where the lips make contact but also impairs the lasting property of the coloring material on the lips. Accordingly, for lip cosmetics, a composition that has an effect of minimizing color transfer is demanded, and studies have been conducted for various compositions having such an effect.

For example, a composition containing a volatile solvent has been studied. A composition containing a volatile solvent allows a non-volatile component that contains a coloring material to stick onto the lips upon volatilization of the volatile solvent so that color transfer does not easily occur, but it has drawbacks like likeliness of causing dryness of the lips after wearing it for a while, difficulty in giving satisfactory shine, and having limitation of the container that can be used.

Meanwhile, compositions that do not contain a volatile solvent have also been studied (for example, Patent literatures 1, 2 and 3). For example, Patent literature 1 proposes a solid cosmetic for lips comprising two types of oil agents, namely, an adhesive oil and an oozing oil, and a wax that disperses at 90°C and solidifies at 25°C when mixed with the oozing oil, wherein the adhesive oil and the oozing oil are separated when mixed at 25°C such that the adhesive oil is dispersed in the oozing oil. This solid cosmetic for lips has a structure in which an inner phase containing the adhesive oil is dispersed in an outer phase containing the oozing oil and the wax, where the solid structure of the wax disintegrates when it is applied onto the lips, and sticks onto the lips in two separating layers, namely, a layer closer to the lips containing the adhesive oil and the wax, and a layer containing the oozing oil. In this composition, the coloring material is likely to be wetted by the adhesive oil to be encapsulated therein. Therefore, even when the lips touch a cup or the like, the coloring material hardly sticks to the cup while the oozing oil in the surface layer sticks to the cup, thereby preventing color transfer.

### CITATION LIST

### Patent Literatures

Patent literature 1: Japanese Patent Publication No. 5926896
Patent literature 2: International Publication WO 2012/165130
Patent literature 3: International Publication WO 2011/071148

### SUMMARY OF INVENTION

### Technical Problem

Since, however, the wax is dispersed in the oozing oil in the conventional art system, the oozing oil needs to disintegrate the fine solid structure of the wax before coming out onto the surface, and thus separation into layers upon application onto the lips takes time. Moreover, since the coloring material is released from the inner phase upon application onto the lips, the color is ununiformly placed onto the lips and thus likely to be uneven.

Under such circumstances, there still remains a need for providing a lip cosmetic composition that has a smooth feeling of use, that gives shine, that has no limitation of the container used, and that allows even color application with little color transfer.

### Solution to Problem

The present invention relates to a lip cosmetic composition and use thereof as recited below. The present invention is defined by the claims.
[1] A lip cosmetic composition comprising an inner phase and an outer phase,
   wherein
   the inner phase comprises:
      A) a low-viscosity oil agent that does not dissolve a solidifier, wherein said low-viscosity oil agent as component A comprises diphenyl dimethicone represented by Formula (1): 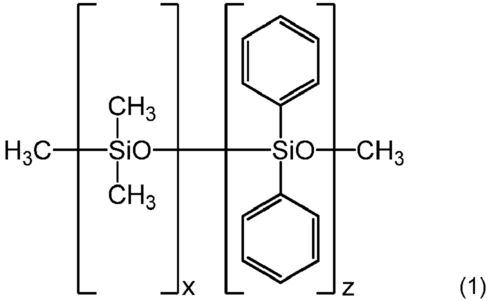
   wherein x + z is in the range of 2-50, and the siloxane units may have a random copolymer or block copolymer arrangement in the diphenyl dimethicone,
   and the outer phase comprises:
      B) a solidifier, wherein the solidifier as component B is (ethylene/propylene) copolymer and synthetic wax;
      C) a high-viscosity oil agent that dissolves the solidifier, wherein the high-viscosity oil agent as component C is a combination of dipentaerythrityl tetra(hydroxystearate/isostearate) and diisostearyl malate;
      D) a binder oil that dissolves the low-viscosity oil agent as component A at 90°C but not at 25°C, wherein the binder oil as component D is octyldodecanol; and
      E) a coloring material,
   the content of component A is 40-70 mass% to the total mass of the lip cosmetic composition,
   and the total amount of components A, B, C, D and E contained in the lip cosmetic composition is 80 mass% or more,
   wherein the ratio of the content of component A to the content of component C (component A/component C) in the lip cosmetic composition of the present invention is greater than 1.2 (mass basis).
[2] The composition according to [1], wherein the viscosity of component A measured at 25°C is in the range of 5-1000 mm²/s.
[3] The composition according to [1] or [2], wherein the ratio of the content of component A to the content of component C (component A/component C) is greater than 1.5 (mass basis), and the ratio of the content of component D to the content of component C (component D/component C) is greater than 0.1 (mass basis).
[4] The composition according to any one of [1] to [3], wherein components A, B, C and D form a homogeneous system at 90°C.
[5] Use of the composition according to any one of [1] to [4] for improving non-transfer properties of the lip cosmetic composition.

### ADVANTAGEOUS EFFECTS OF INVENTION

The present invention can provide a novel lip cosmetic composition that is free of a volatile oil agent. According to a preferable aspect of the prevent invention, a lip cosmetic can be provided that has smooth feeling of use, that has no limitation of the container that can be used, and that has little color transfer. According to a preferable aspect of the present invention, a lip cosmetic can be provided that, in addition to the above-described characteristics, further gives shine and allows even color application upon application onto the lips.

### DESCRIPTION OF EMBODIMENTS

Hereinafter, the lip cosmetic composition of the present invention and use thereof will be described in detail.

A lip cosmetic composition of the present invention comprises an inner phase and an outer phase, wherein
the inner phase comprises:
   A) a low-viscosity oil agent that does not dissolve a solidifier, wherein said low-viscosity oil agent as component A comprises diphenyl dimethicone represented by Formula (1): 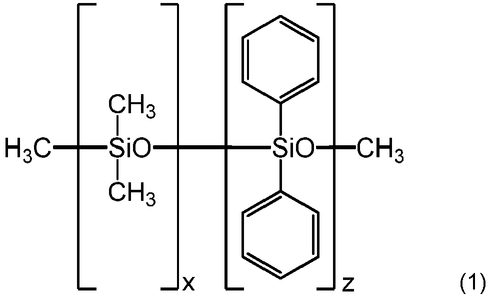
wherein x + z is in the range of 2-50, and the siloxane units may have a random copolymer or block copolymer arrangement in the diphenyl dimethicone,
and the outer phase comprises:
   B) a solidifier, wherein the solidifier as component B is (ethylene/propylene) copolymer and synthetic wax;
   C) a high-viscosity oil agent that dissolves the solidifier, wherein the high-viscosity oil agent as component C is a combination of dipentaerythrityl tetra(hydroxystearate/isostearate) and diisostearyl malate;
   D) a binder oil that dissolves the low-viscosity oil agent as component A at 90°C but not at 25°C, wherein the binder oil as component D is octyldodecanol; and
   E) a coloring material,
the content of component A is 40-70 mass% to the total mass of the lip cosmetic composition,
and the total amount of components A, B, C, D and E contained in the lip cosmetic composition is 80 mass% or more,
wherein the ratio of the content of component A to the content of component C (component A/component C) in the lip cosmetic composition of the present invention is greater than 1.2 (mass basis).

The present inventors conceived of using a low-viscosity oil agent that does not dissolve a solidifier so as to produce a cosmetic composition in which the low-viscosity oil agent is dispersed in a high-viscosity oil agent solidified with the solidifier. If such a cosmetic composition can be obtained, the rate of layer separation upon application onto the lips can be made faster since the solidifier is not dispersed in the low-viscosity oil agent. In addition, since the coloring material is highly compatible with the high-viscosity oil agent and is contained in the outer phase together with the high-viscosity oil agent, the high-viscosity oil agent and the coloring material can easily stick onto the lips upon application onto the lips and leave the color evenly on the lips as the outer phase makes contact with the lips.

In order to produce such a cosmetic composition, a binder oil that binds the low-viscosity oil agent, the high-viscosity oil agent and the solidifier is necessary. Any binder oil that dissolves the low-viscosity oil agent at 90°C but not at 25°C can realize a cosmetic composition having the above-described structure since the low-viscosity oil agent, the high-viscosity oil agent and the solidifier can homogeneously be mixed at 90°C while the inner phase containing the low-viscosity oil agent can be separated from the outer phase containing the high-viscosity oil agent, the solidifier and the binder oil at 25°C. The present invention was accomplished based on these findings.

According to a preferable aspect of the present invention, effects like the followings can be achieved: the rate of layer separation is fast upon application onto the lips since the solidifier is not dispersed in the low-viscosity oil agent; and the high-viscosity oil agent and the coloring material can easily stick onto the lips and leave the color evenly on the lips as the outer phase first makes contact with the lips upon application onto the lips since the coloring material is highly compatible with the high-viscosity oil agent and is contained in the outer phase together with the high-viscosity oil agent.

Hereinafter, each of the components will be described more specifically.

Component A is a low-viscosity oil agent that does not dissolve a solidifier.

The viscosity of component A is not particularly limited as long as it is a liquid oil agent that has lower viscosity than that of a high-viscosity oil agent as component C, the viscosity measured at 25°C is preferably in a range of 5-1000 mm²/s, more preferably 5-600 mm²/s, still more preferably 5-400 mm²/s and particularly preferably 5-200 mm²/s. If the viscosity of the low-viscosity oil agent is within the above-mentioned range, separation into layers upon application of the lip cosmetic composition onto the lips can be made fast.

As disclosed herein, component A contains a silicone oil having a siloxane unit(s) (a) or a siloxane unit(s) (b) represented by the following formula for 50% or more of the total number of siloxane units contained in component A: 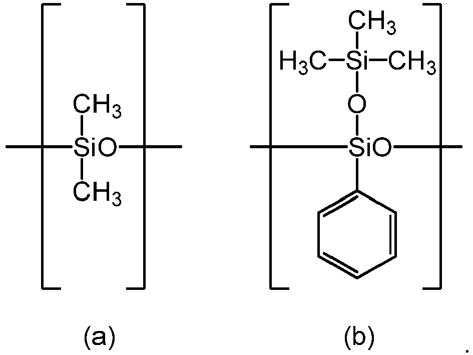 The siloxane unit(s) (a) or the siloxane unit(s) (b) is more preferably 60 % or more, and still more preferably 80 % or more of the total number of siloxane units contained in component A.

The silicone oil having the siloxane unit(s) (a) or the siloxane unit(s) (b) for 50% or more of the total number of siloxane units contained in component A can form an inner phase of the lip cosmetic composition of the present invention without dissolving the solidifier.

The silicone oil used as component A may contain a combination of the siloxane unit (a) and the siloxane unit (b). For example, it may have the siloxane unit(s) (a) for 50% or more of the total number of siloxane units and the siloxane unit(s) (b) for less than 50% of the total number of siloxane units. Alternatively, it may have the siloxane unit(s) (b) for 50% or more of the total number of siloxane units and the siloxane unit(s) (a) for less than 50% of the total number of siloxane units.

As disclosed herein, component A may further contain a siloxane unit (c) represented by the following formula: 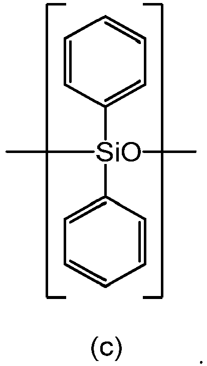 In addition to at least one of the siloxane unit (a) and the siloxane unit (b), it may further contain the siloxane unit (c) to enhance the shine effect of the silicone oil. When a high-shine lip cosmetic is required, a silicone oil containing the siloxane unit (c) is preferably used as component A.

Said low-viscosity oil agent as component A according to the invention comprises diphenyl dimethicone represented by Formula (1): 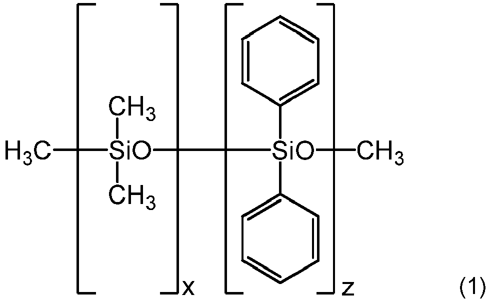 wherein, x + z is in a range of 2-50, and the siloxane units may have a random copolymer or a block copolymer arrangement.

The number x of the repeating siloxane unit (a) is preferably in a range of 1-40, more preferably 1-20 and still more preferably 3-10.

The number z of the repeating siloxane unit (c) is preferably in a range of 1-40, more preferably 1-20 and still more preferably 1-8.

The total number (x + z) of the repeating siloxane units (a) and (c) is more preferably 2-20, and still more preferably 3-12.

As disclosed herein, phenyl trimethyl dimethicone represented by Formula (2) is contained as component A: 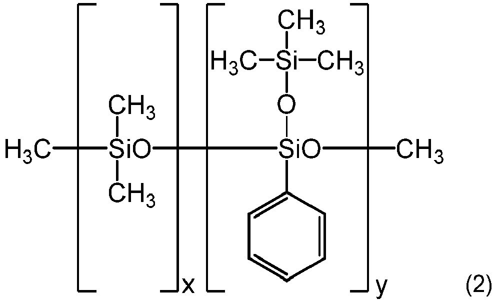 wherein, x + y is in a range of 2-100, and the siloxane units may have a random copolymer or a block copolymer arrangement.

The number x of the repeating siloxane unit (a) is preferably in a range of 1-80, more preferably 1-20 and still more preferably 1-10.

The number y of the repeating siloxane unit (b) is preferably in a range of 1-50, more preferably 1-20, and still more preferably 1-8.

The total number (x + y) of the repeating units x and y of the siloxane units (a) and (b) is more preferably 2-100, and still more preferably 3-20.

As disclosed herein, diphenyl phenyl trimethyl siloxane represented by Formula (3) is contained as component A: 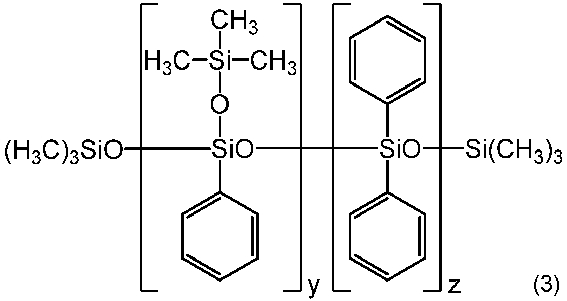 wherein, y + z is in a range of 2-21 provided that y > z, and the siloxane units may have a random copolymer or a block copolymer arrangement.

The number y of the repeating siloxane unit (b) is preferably in a range of 1-20, more preferably 1-10 and still more preferably 1-5.

The number z of the repeating siloxane unit (c) is preferably in a range of 1-20, more preferably 1-10 and still more preferably 1-5.

The total number (y + z) of the repeating siloxane units (b) and (c) is more preferably 2-20, and still more preferably 3-10.

As disclosed herein, component A may be dimethicone or phenyl trimethicone represented by Formula (4) or (5), which has the siloxane unit (a) or the siloxane unit (b) alone: 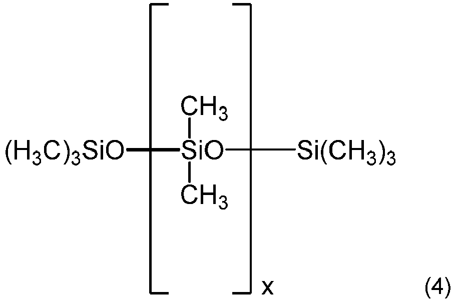 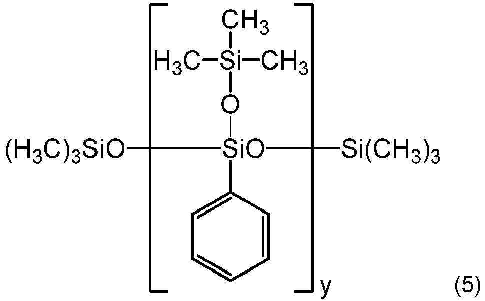 wherein, x is in a range of 1-60 and y is in a range of 1-10.

The number x of the repeating unit is more preferably in a range of 1-20, and still more preferably 1-10.

The number y of the repeating unit is more preferably in a range of 1-6, and still more preferably 1-3.

One or two or more types of them can be used in combination as component A.

The content of component A according to the invention is 40-70 mass% to the total mass of the lip cosmetic composition.

The solidifier as component B used in the lip cosmetic composition of the present invention is not particularly limited as long as it is one that is generally used for lip cosmetics. The solidifier as component B is preferably one that is dissolved in a mixed solution of the high-viscosity oil agent as component C and the binder oil as component D at a melting point but not in the low-viscosity oil agent as component A, and that solidifies at room temperature (25°C).

The solidifier as component B according to the invention is (ethylene/propylene) copolymer and synthetic wax.

As disclosed herein, examples of the solidifier as component B include solid waxes (for example, polyethylene wax, bead wax, microcrystalline wax, solid paraffin, Japan wax, carnauba wax, candelilla wax, ozokerite, ceresin, rice bran wax, spermaceti, bees wax, shellac wax), metallic soaps (for example, calcium stearate, magnesium stearate, aluminum stearate, zinc stearate, zinc laurate, zinc myristate); dextrin derivatives (for example, dextrin palmitate, dextrin palmitate/ethylhexanoate, dextrin palmitate/hexyldecanoate, dextrin myristate, dextrin isostearate); inulin derivatives (for example, inulin stearate); glycerin fatty acid ester (for example, glyceryl behenate/eicosadioate, poly glyceryl-10 behenate/eicosadioate, glyceryl tribehenate/isostearate/eicosandioate); and amino acid derivatives (for example, dibutyl ethylhexanoyl glutamide, dibutyl lauroyl glutamide).

One or two or more types of them can be used in combination as component B.

The content of component B is preferably 2-20 mass%, more preferably 2-15 mass% and still more preferably 4-10 to the total mass of the lip cosmetic composition.

The high-viscosity oil agent as component C used for the lip cosmetic composition of the present invention is not particularly limited as long as it is a liquid or semi-solid (paste) oil agent that dissolves the solidifier as component B, and that has higher viscosity than that of the low-viscosity oil agent as component A. An oil agent that is incompatible with the low-viscosity oil agent as component A, that completely dissolves the solidifier as component B at a melting point, and that is excellent in adhesiveness onto the lips is preferable.

The high-viscosity oil agent as component C according to the invention is a combination of dipentaerythrityl tetra(hydroxystearate/isostearate) and diisostearyl malate.

As disclosed herein, examples of the high-viscosity oil agent as component C include dipentaerythrityl tetra(hydroxystearate/isostearate), lanolin ester, phytostearyl macadamia nut fatty acid, vaseline, diisostearyl malate, cholesterol derivatives (for example, cholesteryl stearate, fatty acid C10-30 cholesterol/lanosterol esters), polyglyceryl-5 triisostearate, hydrogenated polyisobutene and phytosteryl/behenyl dimer dilinoleate.

One or two or more types of them can be used in combination as component C.

The content of component C is preferably 10-50 mass%, more preferably 20-30 mass% and still more preferably 15-25 to the total mass of the lip cosmetic composition.

The binder oil as component D used for the lip cosmetic composition of the present invention dissolves the low-viscosity oil agent as component A at 90°C but not at 25°C.

The binder oil as component D according to the invention is octyldodecanol.

As disclosed herein, examples of the binder oil as component D include octyldodecanol, octyldodecyl stearoyl stearate, octyldodecyl isostearate, dioctyldodecyl lauroyl glutamate, dioctyldodecyl stearoyl glutamate, bis(hexyldecyl/octyldodecyl) lauroyl glutamate, octyldodecyl ricinoleate, squalane, trimethylolpropane triisostearate, and polyglyceryl-2 tetraisostearate.

One or two or more types of them can be used in combination as component D.

The content of component D is preferably 5-40 mass%, more preferably 7-30 mass%, and still more preferably 10-20 to the total mass of the lip cosmetic composition.

The coloring material as component E used for the lip cosmetic composition of the present invention is not particularly limited as long as it is one that is generally used for a lip cosmetic. The coloring material as component E comprise, for example, at least one coloring material selected from water-soluble or oil-soluble coloring agents, the fillers having the effect of coloring and/or opacifying the composition and/or coloring the lips, such as pigments, mother-of-pearls, lacquers (water-soluble coloring agents adsorbed on an inert mineral support) and mixtures thereof.

These coloring materials may optionally be treated at the surface by a hydrophobic agent such as silanes, silicones, fatty acid soaps, C₉-C₁₅ fluoroalcohol phosphates, acrylate/dimethicone copolymers, mixed C₉-C₁₅ fluoroalcohol phosphates/silicone copolymers, lecithins, carnauba wax, polyethylene, chitosan and amino acids optionally acylated such as lauroyl lysine, disodium stearoyl glutamate and aluminum acyl glutamate. The pigments may either be mineral or organic, natural or synthetic.

Examples of pigments include iron, titanium or zinc oxides, as well as composite pigments and goniochromatic, pearlescent, interferential, photochromic or thermochromic pigments. Specifically, examples include hemispherical composite pigments made from cross-linked poly(methylacrylic acid) methyl ester and from organic coloring agents. Such composite pigments are notably marketed by DAITO KASEI.

The mothers-of-pearls may be selected from those generally used in lip cosmetics, such as mica/titanium dioxide products. Alternatively, these may be mothers-of-pearls based on mica/silica/titanium dioxide, based on synthetic fluorphlogopite/titanium dioxide (SUNSHINE (registered trademark) from MAPRECOS), calcium sodium borosilicate/titanium dioxide (REFLECKS (registered trademark) from ENGELHARD) and calcium aluminum borosilicate/silica/titanium dioxide (RONASTAR (registered trademark) from MERCK).

One or two or more types of them can be used in combination as component E.

The content of component E is preferably 0.2-20 mass%, more preferably 1-15 mass% and still more preferably 3-6 to the total mass of the lip cosmetic composition.

In the lip cosmetic composition according to the present invention, the total amount of components A, B, C, D and E is 80 mass% or more, preferably 85 mass% or more, more preferably 90 mass% or more, and still more preferably 95 mass% or more. As long as the contents of the essential components are in the above-mentioned range, the non-transfer effect can be achieved.

According to the present invention, the ratio of the content of component A to the content of component C (component A/component C) in the lip cosmetic composition of the present invention is greater than 1.2 (mass basis), preferably greater than 1.5, and particularly preferably greater than 2.0. Also, component A/component C is preferably less than 10, more preferably less than 5, even more preferably less than 4, and particularly preferably less than 2.5. As long as the ratio of component A and component C is in the above-mentioned range, separation into layers for obtaining the non-transfer effect can rapidly take place.

Furthermore, the ratio of the content of component D to the content of component C (component D/component C) is preferably greater than 0.1 (mass basis), more preferably greater than 0.2, still more preferably greater than 0.5 and particularly preferably greater than 0.7. Also, component D/component C is preferably less than 5, more preferably less than 3, even more preferably less than 2, and particularly preferably less than 1. As long as the ratio of component C and component D is in the above-mentioned range, a homogeneous system can be formed at 90°C.

Moreover, in the lip cosmetic composition according to the present invention, components A, B, C and D are preferably those that form a homogeneous system at 90°C. If these components can form a homogeneous system at 90°C upon production of the lip cosmetic composition, a lip cosmetic composition that is highly homogeneous even in a solid state can be obtained.

The lip cosmetic composition according to the present invention may also contain other oil agent. Examples include, natural oils, hydrocarbon oils, ester oils, higher alcohols and fatty acids. Depending on the combination of components A and B as well as component C or D that are used, these oil agents may be used as component C or D in some cases.

Examples of the natural oils include avocado oil, camellia oil, turtle oil, macadamia nut oil, corn oil, mink oil, olive oil, rapeseed oil, egg yolk oil, sesame oil, persic oil, wheat germ oil, camellia oleifera seed oil, castor oil, flaxseed oil, safflower oil, cottonseed oil, perilla oil, soybean oil, peanut oil, tea seed oil, kaya oil, rice bran oil, Chinese tung oil, Japanese tung oil, jojoba oil, jojoba seed oil, rice germ oil, meadowfoam oil, coconut oil, palm oil, palm kernel oil, fatty acids, namely, linoleic acid, linolenic acid, caprylic acid, capric acid, isostearic acid, hydrogenated coconut fatty acid, and coco-caprylate/caprate.

Examples of hydrocarbon oils include alkanes, namely, paraffin (undecane, tridecane, light paraffin, liquid paraffin), isoparaffin (isodecane, isododecane, isohexadecane, light isoparaffin, hydrogenated polyisobutene), hydrogenated polydecene, squalane, pristane, squalene, cycloparaffin, and coconut alkanes.

Examples of ester oils include isononyl isononanoate, isodecyl isononanoate, isotridecyl isononanoate, ethylhexyl isononanoate, neopentyl glycol diisononanoate, tricyclodecanemethyl isononanoate, cetyl ethylhexanoate, hexyldecyl ethylhexanoate, neopentyl glycol diethylhexanoate, trimethylolpropane triethylhexanoate, isostearyl palmitate, isopropyl palmitate, trimethylolpropane triethylhexanoate, trimethylolpropane triisostearate, glyceryl tri(2-ethylhexanoate) (triethyl hexanoin), pentaerythrityl tetraethylhexanoate, isostearyl myristate, isopropyl myristate, isotridecyl myristate, octyldodecyl myristate, isocetyl myristate, dihexyldecyl myristate, diethyl sebacate, diethylhexyl sebacate, diisopropyl sebacate, diisopropyl adipate, diisobutyl adipate, dihexyldecyl adipate, isodecyl neopentanoate, hexyl laurate, distearyl malate, diisostearyl malate, isocetyl stearate, butyl stearate, 2-ethylhexyl stearate, hexyldecyl dimethyloctanoate, decyl oleate, octyldodecyl erucate, isobutyl isostearate, isocetyl isostearate, ethyl isostearate, isopropyl isostearate, hexyldecyl isostearate, isostearyl isostearate, glyceryl tri(caprylate/caprate), glyceryl tricaprylate, diethylhexyl succinate, bis-ethoxydiglycol succinate, neopentyl glycol diethylhexanoate, neopentyl glycol dicaprate, and isostearyl neopentanoate.

The higher alcohol is preferably one with a carbon number of 22 or less, and more preferably one with a carbon number of 8-18. Examples include isostearyl alcohol, octyldodecanol, oleyl alcohol, decyltetradecanol and hexyldecanol.

The fatty acid is preferably one with a carbon number of 22 or less, and more preferably one with a carbon number of 6-20. Examples include oleic acid, isostearic acid, linoleic acid, linolenic acid, caprylic acid, capric acid, and hydrogenated coconut fatty acid.

According to a preferable aspect of the prevent invention, the lip cosmetic composition according to the present invention can be used to improve the non-transfer properties of the lip cosmetics. Although it is acceptable to include small amounts of volatile oil agent(s) in the lip cosmetic composition of the present invention, it is preferred that component A, component C, component D, and other oily components be non-volatile oils. The allowable content of the volatile oil agent is preferably 5% by mass or less, more preferably 2% by mass or less, even more preferably 1% by mass or less, and most preferably it does not contain the volatile oil agent. According to a preferable aspect of the prevent invention,, since the lip cosmetic composition according to the present invention is free of a volatile oil agent, it has smooth feeling upon use, with shine and without limitation of the container that can be used. Furthermore, since the lip cosmetic composition according to the present invention has good layer separation property upon application onto the lips and the coloring material is contained in the outer phase together with the high-viscosity oil agent, the coloring material can easily stick onto the lips upon application onto the lips and leaves the color evenly on the lips.

The lip cosmetic composition of the present invention can be produced by the following steps:
(a) heating components A, B, C, D and E in a container at a temperature that allows components A, B, C and D to form a homogeneous system and mixing the resultant until they are homogeneous; and
(b) subjecting the composition obtained in the above step to defoamation, if necessary, and filling the resultant into a predetermined container while maintaining the above-mentioned temperature, and then cooling.

The temperature of components A, B, C and D to form a homogeneous system in Step (a) is, for example, 70-120 °C, preferably 80-100 °C and more preferably 90°C.

While the cooling temperature in Step (b) is not particularly limited as long as it is a temperature that allows the resulting composition to be in a solid state, it is, for example, -30-30 °C, preferably -20-0 °C and more preferably -20- -10 °C.

As described above, the lip cosmetic composition of the present invention can be produced by a simple method without a particular procedure.

The lip cosmetic composition according to the present invention may contain components other than the above-described components within a range that does not impair the object and the effect of the present invention. For example, it may contain an component that can be added to a skin care composition such as a pharmaceutical product, a quasi-drug or cosmetics.

As the optional component(s) usable in the present invention, for example, powder component(s), surfactant(s), cosurfactant(s), moisturizer(s), film agent(s), thickener(s), gelatinizer(s), inorganic mineral(s), sequestering agent(s), polyhydric alcohol(s), monosaccharide(s), oligosaccharide(s), amino acid(s), plant extract(s), organic amine(s), polymer emulsion(s), antioxidant(s), oxidization prevention assistant(s), skin nutritional supplement(s), vitamin(s), bloodstream accelerant(s), sterilizer(s), antiphlogistic (antiinflammation) agent(s), cell (skin) activation agent(s), keratolytic agent(s), tonic(s), astrictive(s), whitening agent(s), UV absorber(s), fading inhibitor(s), preservative(s), buffer(s) and/or fragrance(s) can be appropriately contained as needed. These optional components can be appropriately selected depending on the formulation form and usage, etc. to be aimed at.

Examples of the powder components include inorganic powders (for example, talc, kaolin, mica, sericite, muscovite, phlogopite, synthetic mica, deep red mica, biotite, vermiculite, magnesium carbonate, calcium carbonate, aluminum silicate, barium silicate, calcium silicate, magnesium silicate, strontium silicate, tungstic acid metal salt, silica, zeolite, barium sulfate, magnesium sulfate, burnt calcium sulfate (plaster), calcium phosphate, fluorine apatite, hydroxyapatite, ceramic powders, metallic soaps (for example, zinc myristate, calcium palmitate, aluminum stearate, magnesium stearate), boron nitride); organic powders (for example, polyamide resin powder (nylon powder), polyethylene powder, polymethyl methacrylate powder, polystyrene powder, co-polymer resin powder of styrene and acrylic acid, benzoguanamine resin powder, polytetrafluoroethylene powder, cellulose powder); metallic powder pigments (for example, aluminum powder, copper powder), organic pigments such as zirconium, barium or aluminum lakes; and natural pigments (for example, chlorophyll, β-carotene). Here, the powder components may be subjected to a hydrophobic treatment.

The surfactants may include anionic surfactants, cationic surfactants, ampholytic surfactants, lipophilic nonionic surfactants and hydrophilic nonionic surfactants.

Examples of the anionic surfactant include fatty acid soaps (for example, sodium laurate and sodium palmitate); higher alkyl sulfate salts (for example, sodium lauryl sulfate and potassium lauryl sulfate); alkylether sulfate salts (for example, triethanolamine POE-lauryl sulfate and POE-sodium lauryl sulfate); N-acyl sarcosine acids (for example, sodium lauroylsarcosinate); higher fatty acid amide sulfonates (for example, sodium N-myristoyl-N-methyl taurate, sodium cocoyl methyl tauride and sodium lauryl methyltauride); phosphate salts (sodium POE-oleyl ether phosphate, a POE-stearyl ether phosphoric acid, etc.); sulfosuccinates (for example, sodium di-2-ethylhexylsulfosuccinate, sodium monolauroyl monoethanolamide polyoxyethylene sulfosuccinate and sodium lauryl polypropylene glycol sulfosuccinate); alkyl benzenesulfonates (for example, linear sodium dodecylbenzenesulfonate, linear triethanolamine dodecylbenzenesulfonate and a linear dodecylbenzenesulfonic acid); higher fatty acid ester sulfate salts (for example, sodium cocomonoglyceride sulfate); N-acyl glutamates (for example, monosodium N-lauroyl glutamate, disodium N-stearoyl glutamate and monosodium N-myristoyl-L-glutamate); sulfated oils (for example, Turkey red oil); POE-alkylether carboxylic acids; POE-alkylallylether carboxylates; α-olefin sulfonates; higher fatty acid ester sulfonates; secondary alcohol sulfate ester salts; higher fatty acid alkylolamide sulfate ester salts; sodium lauroyl monoethanolamide succinates; ditriethanolamine N-palmitoyl aspartate; and sodium casein.

Examples of the cationic surfactant include alkyltrimethylammonium salts (for example, stearyltrimethylammonium chloride and lauryltrimethylammonium chloride); alkylpyridinium salts (for example, cetylpyridinium chloride); a chloride distearyldimethylammonium dialkyldimethylammonium salt; poly(N,N'-dimethyl-3,5-methylene piperidinium) chloride; alkyl quaternary ammonium salts; alkyldimethylbenzylammonium salts; alkylisoquinolinium salts; dialkylmorpholium salts; POE-alkylamine; alkylamine salts; polyamine fatty acid derivatives; amyl alcohol fatty acid derivatives; benzalkonium chloride; and benzethonium chloride.

Examples of the ampholytic surfactant include imidazoline-based ampholytic surfactants (for example, sodium 2-undecyl-N,N,N-(hydroxyethylcarboxymethyl)-2-imidazoline; and a 2-cocoyl-2-imidazolinium hydroxide-1-carboxyethyloxy disodium salt); and betaine-based surfactants (for example, 2-heptadecyl-N-carboxymethyl-N-hydroxyethyl imidazolinium betaine, lauryldimethylamino acetate betaine, alkyl betaine, amide betaine, and sulfobetaine).

Examples of the lipophilic nonionic surfactant include sorbitan fatty acid esters, such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate and diglycerol sorbitan tetra-2-ethylhexylate; glyceryl polyglyceryl fatty acids, such as glyceryl mono-cottonseed oil fatty acid, glyceryl monoerucate, glyceryl sesquioleate, glyceryl monostearate, glyceryl α,α'-oleate pyroglutamate, and glyceryl monostearate malate; propylene glycol fatty acid esters such as monostearate propylene glycol; a hydrogenated castor oil derivative; a glycerin alkyl ether; and steareth-2.

Examples of the hydrophilic nonionic surfactant include POE-sorbitan fatty acid esters, such as POE-sorbitan monooleate, POE-sorbitan monostearate, POE-sorbitan monooleate and POE-sorbitan tetraoleate; POE sorbitol fatty acid esters, such as POE-sorbitol monolaurate, POE-sorbitol monooleate, POE-sorbitol pentaoleate and POE-sorbitol monostearate; POE-glycerin fatty acid esters, such as POE-glycerin monostearate, POE-glycerin monoisostearate and POE-glycerin triisostearate; POE-fatty acid esters, such as POE-monooleate, POE-distearate, POE-monodioleate and ethylene glycol distearate; POE-alkyl ethers, such as POE-lauryl ether, POE-oleyl ether, POE-stearyl ether, POE-behenyl ether, POE-2-octyldodecyl ether and POE-cholestanol ether; Pluronic type surfactants (e.g., Pluronic); POE-POP-alkyl ethers, such as POE-POP-cetyl ether, POE-POP-2-decyltetradecyl ether, POE-POP-monobutyl ether, POE-POP-hydrogenated lanolin and POE-POP-glycerin ether; and steareth-21.

An oil-soluble thickener can be used as the thickener. For example, silicone derivatives having a siloxane bond in the backbone and having silicone or polyoxyethylene cross-linked or long-chain hydrocarbon introduced to a side chain (for example, a (dimethicone/vinyl dimethicone) cross polymer, a (dimethicone/phenyl vinyl dimethicone) cross polymer, a (vinyl dimethicone/lauryl dimethicone) cross polymer); aluminum salts of double-chain long-chain alkyl phosphoric acid esters (for example, aluminum salt of dihexadecyl phosphate); lecithins; amorphous silicas; 12-hydroxystearic acid can be used.

Clay minerals such as organically-modified clay minerals may also be used as the thickener.

Examples of the polyhydric alcohol include a dihydric alcohol, such as ethylene glycol, propylene glycol, pentylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol and octylene glycol; a trihydric alcohol, such as glycerin and trimethylolpropane; a tetrahydric alcohol such as pentaerythritol (e.g., 1,2,6-hexanetriol); a pentahydric alcohol such as xylitol; a hexahydric alcohol, such as sorbitol and mannitol; a polyhydric alcohol polymer, such as diethylene glycol, dipropylene glycol, triethylene glycol, polypropylene glycol and tetraethylene glycol; dihydric alcohol alkyl ethers, such as ethylene glycol monomethyl ether and ethylene glycol monoethyl ether; dihydric alcohol alkyl ethers, such as diethylene glycol monomethyl ether, diethylene glycol monoethyl ether and diethylene glycol monobutyl ether; a dihydric alcohol ether ester, such as ethylene glycol monomethyl ether acetate and ethylene glycol monoethyl ether acetate; a glycerol monoalkyl ether, such as chimyl alcohol, selachyl alcohol and batyl alcohol; and a sugar alcohol, such as sorbitol, maltitol, maltotriose, mannitol, sucrose, erythritol, glucose, fructose, starch sugar, maltose, xylitose, and a reduced alcohol of a starch sugar.

Examples of the antioxidants include ascorbic acid and derivatives thereof such as ascorbyl palmitate and ascorbyl tetraisopalmitate, ascorbyl glucoside, magnesium ascorbyl phosphate, sodium ascorbyl phosphate, and ascorbyl sorbate; tocopherol and derivatives thereof, such as tocopheryl acetate, tocopheryl sorbate, and other esters of tocopherol; dibutyl hydroxytoluene (BHT) and butylated hydroxyanisole (BHA); gallic acid ester.

Furthermore, the composition of the present invention may also include organic and/or inorganic sunscreens.

Examples of the organic sunscreens include dibenzoylmethane derivatives such as butyl methoxydibenzoylmethane (for example, a product commercially available from HOFFMANN LA ROCHE under the trade name of Parsol 1789); cinnamic acid derivatives such as octyl methoxycinnamate (for example, a product commercially available from HOFFMANN LA ROCHE under the trade name of Parsol MCX); salicylates; para-aminobenzoic acids; β, β'-diphenylacrylate derivatives; benzophenone derivatives; benzylidenecamphor derivatives such as terephtalylidene dicamphor sulphonic acid; phenylbenzimidazole derivatives; triazine derivatives; phenylbenzotriazole derivatives; and anthranilic acid derivatives, all of which may be coated or encapsulated.

Examples of the inorganic sunscreens include pigments and nanopigments formed from coated or uncoated metal oxides. Examples of the nanopigments include titanium oxide, iron oxide, zinc oxide, zirconium oxide and cerium oxide nanopigments, which are all well-known as UV photoprotective agents.

Examples of the antiseptic agent include p-oxybenzoate ester (e.g., methylparaben and propylparaben) and phenoxyethanol.

In addition, as an optional component to be used in the composition of the present invention, those mentioned in the International Cosmetic Component Dictionary and Handbook, 13th Edition, 2010, published by the Personal Care Products Council, can be used.

The amounts of these optional components contained are not particularly limited as long as the optional components are in a range which does not impair the object of the present invention.

The lip cosmetic composition according to the present invention can favorably be used in the fields of pharmaceutical products, quasi-drugs, cosmetics and the like.

The product form of the lip cosmetic composition according to the present invention can arbitrarily be selected. For example, it can be used as lipsticks (stick type, cream type, liquid type, etc.), lip gloss, lip liners, and lip balm.

### EXAMPLES

Hereinafter, the present invention will be described by way of Examples and Comparative examples, although the present invention should not be limited to these examples. Unless otherwise specified, the composition ratios in the tables are based on mass ratio (unit: g).

### <Compatibility test 1>

A compatibility test was performed to find a low-viscosity oil agent that does not dissolve a solidifier.

Preparation process: The respective oil agent and the solidifier were heated at 90°C, mixed by stirring to observe the mixed state of the oil when left to stand. Those having a boundary with two uniformly separated layers was evaluated as "insoluble". As shown in Table 1, component A was confirmed not to dissolve the solidifier.

**Table 1**

| Name of component | | Test example 1-1 | Test example 1-2 | Test example 1-3 | Test example 1-4 | Test example 1-5 | Comparative example 1-6 | Comparative example 1-7 |
|---|---|---|---|---|---|---|---|---|
| Component A | Diphenyl dimethicone* 1 | 80 | | 80 | | | | |
| | Diphenyl dimethicone*2 | | 80 | | | | | |
| | Dimethicone*3 | | | | 80 | | | |
| | Dimethicone*4 | | | | | 80 | | |
| Comparative component | Diphenylsiloxy phenyl trimethicone*5 | | | | | | 80 | |
| | Trimethyl pentaphenyl trisiloxane*6 | | | | | | | 80 |
| Component B | Solidifier*7 | 20 | 20 | 20 | 20 | 20 | 20 | 20 |
| Compatibility at 90°C | | Insoluble | Insoluble | Insoluble | Insoluble | Insoluble | Soluble | Soluble |

| | | | | | | | | |
|---|---|---|---|---|---|---|---|---|
| *1 Silicone oil KF-53 (from Shin-Etsu Chemical) *2 Silicone oil KF-54 (from Shin-Etsu Chemical) *3 Silicone oil KF-96-10cs (from Shin-Etsu Chemical) *4 Silicone oil KF-96-20cs (from Shin-Etsu Chemical) *5 Silicone oil KF-56A (from Shin-Etsu Chemical) (the compound of y<z in Formula (3), which does not correspond to component A) *6 Silicone oil PH-1555 (from Dow Corning Toray) *7 Solidifier LIP WAX PZ-80-20 (Ethylene/propylene) copolymer, synthetic wax (from INA Trading) | | | | | | | | |

### <Compatibility test 2>

A compatibility test was performed to find a binder oil that is homogeneously dissolved at 90°C and separated at 25°C with a low-viscosity oil agent that does not dissolve a solidifier at 90°C in the absence of a binder oil.

Preparation process: The respective oil agents were heated at 90°C and mixed by stirring to observe the mixed state of the oil when left to stand and the mixed state of this oil mixture at 25°C. Those having a boundary with two uniformly separated layers was evaluated as "separated", and a translucent state or a transparent compatible state without a boundary was evaluated as "homogeneous". As shown in Table 2, component D was in a homogeneous state with component A at 90°C and separated therefrom at 25°C, confirming that it was effective as a binder oil.

**Table 2**

| Name of components | | Test example 2-1 | Test example 2-2 | Test example 2-3 | Test example 2-4 | Test example 2-5 | Comparative example 2-6 | Comparative example 2-7 |
|---|---|---|---|---|---|---|---|---|
| Component A | Diphenyl dimethicone* 1 | 50 | | 50 | | | | 50 |
| | Diphenyl dimethicone*2 | | 50 | | | | | |
| | Dimethicone*3 | | | | 50 | | | |
| | Dimethicone*4 | | | | | 50 | 50 | |
| Component D | Octyldodecanol | 50 | 50 | | 50 | 50 | | |
| | Trimethylolpropane triisostearate | | | 50 | | | | |
| Comparative Component | Triethyl hexanoin | | | | | | 50 | |
| | Glyceryl tri(caprylate/caprate) | | | | | | | 50 |
| Compatibility at 90°C | | Homogeneous | Homogeneous | Homogeneous | Homogeneous | Homogeneous | Homogeneous | Homogeneous |
| Compatibility at 25°C | | Separated | Separated | Separated | Separated | Separated | Homogeneous | Homogeneous |

| | | | | | | | | |
|---|---|---|---|---|---|---|---|---|
| *1 Silicone oil KF-53 (from Shin-Etsu Chemical) *2 Silicone oil KF-54 (from Shin-Etsu Chemical) *3 Silicone oil KF-96-10cs (from Shin-Etsu Chemical) *4 Silicone oil KF-96-20cs (from Shin-Etsu Chemical) | | | | | | | | |

### <Compatibility test 3>

In order to find out appropriate proportions of component A that imparts non-transfer and shine effects upon separation, component C as a high-viscosity oil agent that separates from component A, and component D as a binder oil for making component A and component C to form a homogeneous system at 90°C, a mixture of the three components was prepared to observe compatibility at 90°C and separation at 25°C.

Preparation process: The respective oil agents were heated at 90°C and mixed by stirring. Then, the mixed state of the oil when left to stand and the mixed state of this oil mixture at 25°C were evaluated as "separated" for those having a boundary with two uniformly separated layers, and as "homogeneous" for a translucent state or a transparent compatible state without a boundary. A state where it is not clearly separated into two layers but was cloudy and undissolved was evaluated as "cloudy". The results are shown in Table 3.

**Table 3**

| Test | | Test example 3-1 | Test example 3-2 | Test example 3-3 | Test example 3-4 | Test example 3-5 | Test example 3-6 | Test example 3-7 | Test example 3-8 | Comparative example 3-9 |
|---|---|---|---|---|---|---|---|---|---|---|
| Component A | Diphenyl dimethicone* 1 | 10.0 | 12.0 | 14.0 | 16.0 | 18.0 | 20.0 | 22.0 | 24.0 | 26.0 |
| Component C | Dipentaerythrityl tetra (hydroxystearate/isostearate) | 5.0 | 5.0 | 5.0 | 5.0 | 5.0 | 5.0 | 5.0 | 5.0 | 5.0 |
| Component D | Octyldodecanol | 20.0 | 18.0 | 16.0 | 14.0 | 12.0 | 10.0 | 8.0 | 6.0 | 4.0 |
| Compatibility at 90°C | | Homogeneous | Homogeneous | Homogeneous | Homogeneous | Homogeneous | Homogeneous | Homogeneous | Homogeneous | Cloudy |
| Compatibility at 25°C | | Separated | Separated | Separated | Separated | Separated | Separated | Separated | Separated | Separated |

| | | | | | | | | | | |
|---|---|---|---|---|---|---|---|---|---|---|
| *1 Silicone oil KF-53 (from Shin-Etsu Chemical) | | | | | | | | | | |

### <Compatibility test 4>

A simple formulation that does not contain a solidifier and a coloring agent was prepared assuming a lipstick formulation to observe compatibility at 90°C and separation at 25°C.

Preparation process: The respective oil agents were heated at 90°C and mixed by stirring. Then, the mixed state of the oil when left to stand and the mixed state of this oil mixture at 25°C were evaluated as "separated" for those having a boundary with two uniformly separated layers, and as "homogeneous" for a translucent state or a transparent compatible state without a boundary. A state where it is not clearly separated into two layers but was cloudy and undissolved was evaluated as "cloudy". As shown in Table 4, those that presented a homogeneous state at 90°C and separated at 25°C were confirmed to be the blended components of the test example.

**Table 4**

| | | Test example 4-1 | Comparative example 4-2 | Comparative example 4-3 | Comparative example 4-4 | Comparative example 4-5 |
|---|---|---|---|---|---|---|
| Component A | Diphenyl dimethicone*1 | 48 | 24 | 24 | 24 | - |
| Comparative component | Diphenylsiloxy phenyl trimethicone*5 | - | 24 | 24 | 24 | 48 |
| Component C | Dipentaerythrityl tetra (hydroxystearate/ isostearate) | 10 | 10 | 10 | 10 | 10 |
| | Diisostearyl malate | 12 | 12 | 12 | 12 | 8 |
| | Polyglyceryl-5 triisostearate | - | - | 18 | - | - |
| | Hydrogenated polyisobutene | - | - | - | 5 | - |
| | Phytosteryl/behenyl dimer dilinoleate | - | - | - | 13 | - |
| Component D | Octyldodecanol | 18 | 18 | - | - | 18 |
| Compatibility at 90°C | | Homogeneous | Homogeneous | Separated | Homogeneous | Homogeneous |
| Compatibility at 25°C | | Separated | Homogeneous | Separated | Cloudy | Homogeneous |

| | | | | | | |
|---|---|---|---|---|---|---|
| *1 Silicone oil KF-53 (from Shin-Etsu Chemical) *5 Silicone oil KF-56A (from Shin-Etsu Chemical) | | | | | | |

### <Formulation and evaluation of stick type lipsticks>

Based on Compatibility test 4 performed above, stick type lipsticks were prepared to confirm shine, non-transfer effects, evenness of coloring, and feeling of use. The results are shown in Table 5.

### Preparation process of lipsticks:

All components were weighed and thereafter heated at 90°C and then mixed until homogeneous to give an oil-phase mixture.

### Following defoamation, the resultant was filled into a lipstick container at 90°C and cooled to give a stick type lipstick for the test.

### Evaluation methods:

Four-grade evaluation was conducted as follows by five evaluators.
+; Good
0; Moderate
Bad
--; Very bad

**Table 5**

| | | Test example 5-1 | Comparative example 5-2 | Comparative example 5-3 | Comparative example 5-4 | Comparative example 5-5 |
|---|---|---|---|---|---|---|
| Component A | Diphenyl dimethicone* 1 | 48 | 24 | 24 | 24 | - |
| Comparative Component | Diphenylsiloxy phenyl trimethicone*5 | - | 24 | 24 | 24 | 48 |
| Component C | Dipentaerythrityl tetra (hydroxystearate/ isostearate) | 10 | 10 | 10 | 10 | 10 |
| | Diisostearyl malate | 12 | 12 | 12 | 12 | 12 |
| | Polyglyceryl-5 triisostearate | - | - | 18 | - | - |
| | Hydrogenated polyisobutene | - | - | - | 5 | - |
| | Phytosteryl/behenyl dimer dilinoleate | - | - | - | 13 | - |
| Component D | Octyldodecanol | 18 | 18 | - | - | 18 |
| Component B | Solidifier*7 | 8 | 8 | 8 | 8 | 8 |
| Component E | Coloring material*8 | 4 | 4 | 4 | 4 | 4 |
| Shine | | + | - | - | 0 | 0 |
| Non-transfer effect | | + | - | 0 | - | - |
| Evenness of coloring | | + | 0 | - | - | - |
| Feeling of use | | + | 0 | - | 0 | 0 |

| | | | | | | |
|---|---|---|---|---|---|---|
| *1 Silicone oil KF-53 (from Shin-Etsu Chemical) *5 Silicone oil KF-56A (from Shin-Etsu Chemical) *7 Solidifier LIP WAX PZ-80-20: (Ethylene/propylene) copolymer, synthetic wax (from INA Trading) *8 Coloring material Red 202 | | | | | | |

As shown in Table 3, Test example 5-1, namely, the lip cosmetic composition according to the present invention, had high shine, high non-transfer effect, had no color unevenness, was homogeneous and had smooth feeling of use upon application.

On the other hand, Comparative examples did not give good result for any of the evaluation points, and particularly gave bad evaluations as a whole for the evenness of coloring.

### < Formulation and evaluation of cream type lipstick>

A cream type lipstick was prepared as follows to confirm shine, non-transfer effect, evenness of coloring, and feeling of use. The results showed good shine, non-transfer effect and smooth feeling of use upon application, and uniform application with no unevenness.

### Preparation process of lipstick:

All components were weighed and thereafter heated at 90°C and then mixed until homogeneous to give an oil-phase mixture.

Following defoamation, the resultant was filled into a lipstick container at 90°C and cooled to give a cream type lipstick for the test.

**Table 6**

| | | |
|---|---|---|
| Component A | Diphenyl dimethicone*! | 46.0 |
| Component B | LIP WAX PZ-80-20*7 | 5.0 |
| Component C | Dipentaerythrityl tetra(hydroxystearate/isostearate ) | 10.0 |
| | Diisostearyl malate | 14.0 |
| Component D | Octyldodecanol | 18.0 |
| Component E | Red 202 | 4.0 |
| | Pearl pigment GOLD*8 | 0.5 |
| | Pearl pigment RED*9 | 0.5 |
| Optional components | Preservative: phenoxyethanol | 0.8 |
| | Organically-modified clay mineral: disteardimonium hectorite | 1.0 |
| | Fragrance | 0.2 |

| | | |
|---|---|---|
| *1 Silicone oil KF-53 (from Shin-Etsu Chemical) *7 LIP WAX PZ-80-20 (Ethylene/propylene) copolymer and synthetic wax (from INA Trading) *8 Pearl pigment GOLD TIMIRON SUPER GOLD (from Merck) *9 Pearl pigment RED RONASTAR RED (from Merck) | | |

## Claims

1. A lip cosmetic composition comprising an inner phase and an outer phase, wherein
the inner phase comprises:
A) a low-viscosity oil agent that does not dissolve a solidifier, wherein said low-viscosity oil agent as component A comprises diphenyl dimethicone represented by Formula (1): 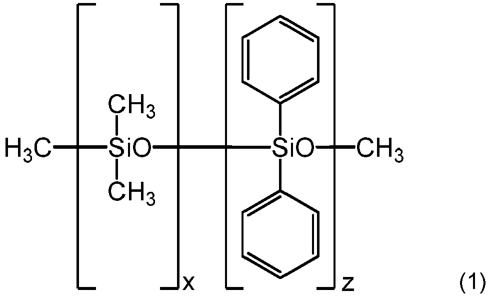
wherein x + z is in the range of 2-50, and the siloxane units may have a random copolymer or block copolymer arrangement in the diphenyl dimethicone,
and the outer phase comprises:
B) a solidifier, wherein the solidifier as component B is (ethylene/propylene) copolymer and synthetic wax;
C) a high-viscosity oil agent that dissolves the solidifier, wherein the high-viscosity oil agent as component C is a combination of dipentaerythrityl tetra(hydroxystearate/isostearate) and diisostearyl malate;
D) a binder oil that dissolves the low-viscosity oil agent as component A at 90°C but not at 25°C, wherein the binder oil as component D is octyldodecanol;
and
E) a coloring material,
the content of component A is 40-70 mass% to the total mass of the lip cosmetic composition,
and the total amount of components A, B, C, D and E contained in the lip cosmetic composition is 80 mass% or more,
wherein the ratio of the content of component A to the content of component C (component A/component C) in the lip cosmetic composition of the present invention is greater than 1.2 (mass basis).

2. The composition according to Claim 1, wherein the viscosity of component A measured at 25°C is in the range of 5-1000 mm²/s.

3. The composition according to Claim 1 or 2, wherein the ratio of the content of component A to the content of component C (component A/component C) is greater than 1.5 (mass basis), and the ratio of the content of component D to the component C (component D/component C) is greater than 0.1 (mass basis).

4. The composition according to any one of Claims 1 to 3, wherein components A, B, C and D form a homogeneous system at 90°C.

5. Use of the composition according to any one of Claims 1 to 4 for improving non-transfer properties of the lip cosmetic composition.

## Patentansprüche

1. Lippenkosmetik-Zusammensetzung, umfassend eine innere Phase und eine äußere Phase, wobei
die innere Phase umfasst:
A) ein niedrigviskoses Ölmittel, das einen Verfestiger nicht auflöst, wobei das niedrigviskose Ölmittel als Komponente A Diphenyldimethicon der Formel (1) umfasst: 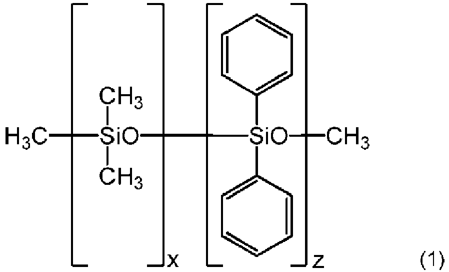
wobei x + z im Bereich von 2-50 liegt und die Siloxan-Einheiten eine zufällige Copolymer- oder Blockcopolymer-Anordnung in dem Diphenyldimethicon aufweisen können,
und die äußere Phase umfasst:
B) einen Verfestiger, wobei der Verfestiger als Komponente B ein (Ethylen/Propylen)-Copolymer und synthetisches Wachs ist;
C) ein hochviskoses Ölmittel, das den Verfestiger auflöst, wobei das hochviskose Ölmittel als Komponente C eine Kombination aus Dipentaerythrityltetra(hydroxystearat/isostearat) und Diisostearylmalat ist;
D) ein Bindemittelöl, das das niedrigviskose Ölmittel als Komponente A bei 90°C, aber nicht bei 25°C auflöst, wobei das Bindemittelöl als Komponente D Octyldodecanol ist; und
E) ein färbendes Material,
der Gehalt der Komponente A 40-70 Massen-% der Gesamtmasse der Lippenkosmetik-Zusammensetzung beträgt,
und die Gesamtmenge der Komponenten A, B, C, D und E, die in der Lippenkosmetik-Zusammensetzung enthalten sind, 80 Massen-% oder mehr beträgt,
wobei das Verhältnis des Gehalts der Komponente A zum Gehalt der Komponente C (Komponente A/Komponente C) in der Lippenkosmetik-Zusammensetzung der vorliegenden Erfindung größer als 1,2 (Massenbasis) ist.

2. Zusammensetzung nach Anspruch 1, wobei die Viskosität der Komponente A, gemessen bei 25°C, im Bereich von 5-1000 mm²/s liegt.

3. Zusammensetzung nach Anspruch 1 oder 2, wobei das Verhältnis des Gehalts der Komponente A zum Gehalt der Komponente C (Komponente A/Komponente C) größer als 1,5 (Massenbasis) ist und das Verhältnis des Gehalts der Komponente D zur Komponente C (Komponente D/Komponente C) größer als 0,1 (Massenbasis) ist.

4. Zusammensetzung nach einem der Ansprüche 1 bis 3, wobei die Komponenten A, B, C und D bei 90°C ein homogenes System bilden.

5. Verwendung der Zusammensetzung nach einem der Ansprüche 1 bis 4 zur Verbesserung der Nicht-Übertragungs-Eigenschaften der Lippenkosmetik-Zusammensetzung.

## Revendications

1. Composition de rouge à lèvres comprenant une phase interne et une phase externe, dans laquelle la phase interne comprend :
A) un agent huileux à faible viscosité qui ne dissout pas un agent solidifiant, dans laquelle ledit agent huileux à faible viscosité en tant que composant A comprend de la diméthicone de diphényle représentée par la Formule (1) : 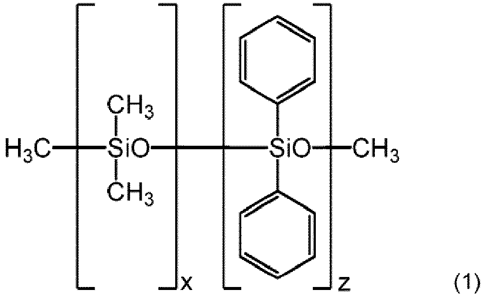
dans laquelle x + z est de 2 à 50, et les motifs siloxane peuvent présenter un agencement de copolymères aléatoires ou de copolymères séquencés dans la diméthicone de diphényle,
et la phase externe comprend :
B) un agent solidifiant, dans laquelle l'agent solidifiant en tant que composant B est un copolymère (éthylène/propylène) et une cire synthétique ;
C) un agent huileux à viscosité élevée qui dissout l'agent solidifiant, dans laquelle l'agent huileux à viscosité élevée en tant que composant C est une combinaison de tétra(hydroxystéarate/isostéarate) dipentaérythrityle et de malate de diisostéaryle ;
D) une huile liante qui dissout l'agent huileux à faible viscosité en tant que composant A à 90 °C mais pas à 25 °C, dans laquelle l'huile liante en tant que composant D est l'octyldodécanol ;
et
E) une matière colorante,
la teneur du composant A est de 40-70 % en masse par rapport à la masse totale de la composition de rouge à lèvres,
et la quantité totale de composants A, B, C, D et E contenus dans la composition de rouge à lèvres est supérieure ou égale à 80 % en masse,
dans laquelle le rapport de la teneur du composant A à la teneur du composant C (composant A/composant C) dans la composition de rouge à lèvres de la présente invention est supérieur à 1,2 (base massique).

2. Composition selon la revendication 1, dans laquelle la viscosité du composant A, mesurée à 25 °C, est de 5 à 1000 mm²/s.

3. Composition selon la revendication 1 ou 2, dans laquelle le rapport de la teneur du composant A à la teneur du composant C (composant A/composant C) est supérieur à 1,5 (base massique), et le rapport de la teneur du composant D au composant C (composant D/composant C) est supérieur à 0,1 (base massique).

4. Composition selon l'une quelconque des revendications 1 à 3, dans laquelle les composants A, B, C et D forment un système homogène à 90 °C.

5. Utilisation de la composition selon l'une quelconque des revendications 1 à 4 pour améliorer les propriétés de non-transfert de la composition de rouge à lèvres.
